# EUROPEAN PATENT APPLICATION

(11) **EP 3 243 394 A1**
(43) Date of publication of application: **15.11.2017**
(21) Application number: 17182707.4
(22) Date of filing: 22.07.2017
(51) Int. Cl.: A24F 47/00, A61M 15/06

(54) **ELECTRONIC CIGARETTE, CONTROL METHOD AND CONTROL SYSTEM HAVING SAME**

(30) Priority: 27.07.2016 CN 201610598336
(71) Applicant: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); CHEN, Hongbin, Shenzhen, Guangdong 518104 (CN); SONG, Shengjia, Shenzhen, Guangdong 518104 (CN); LI, Yuqin, Shenzhen, Guangdong 518104 (CN)
(74) Representative: ProI European Patent Attorneys

(57) **Abstract**

The present disclosure relates to a control method of an electronic cigarette. The electronic cigarette includes the following steps: (a) a mobile device sending a control signal to a server; (b) the server receiving and sending the control signal to the electronic cigarette; and (c) the electronic cigarette receiving the control signal and controlling the working circuit to connect with the power supply based on the control signal.

## Description

### TECHNICAL FIELD

The present invention relates to an electronic cigarette, a control method of the electronic cigarette, and a control system having same.

### BACKGROUND ART

Electronic cigarette are harmful for children. A typical electronic cigarette includes a mechanic child lock. However, children may learn to unlock the mechanic child lock easily.

What are needed, therefore, are an electronic cigarette, a control method of the electronic cigarette, and a control system having same, which can overcome the above shortcoming.

### SUMMARY

The present disclosure relates to a control method of an electronic cigarette. The electronic cigarette includes the following steps: (a) a mobile device sending a control signal to a server; (b) the server receiving and sending the control signal to the electronic cigarette; and (c) the electronic cigarette receiving the control signal and controlling the working circuit to connect with the power supply based on the control signal.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a control system of an electronic cigarette according to an embodiment, the control system including the electronic cigarette, a mobile device, and a server.
FIG. 2 is a cross-sectional view of the electronic cigarette of FIG. 1.
FIG. 3 is a flowchart of a control method of the electronic cigarette of FIG. 1.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

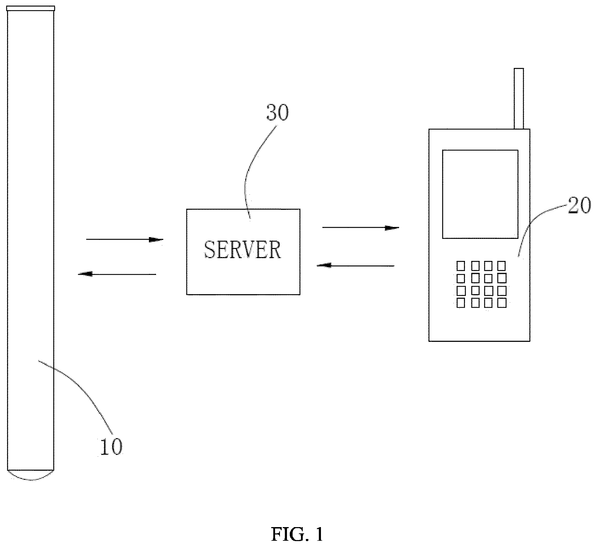

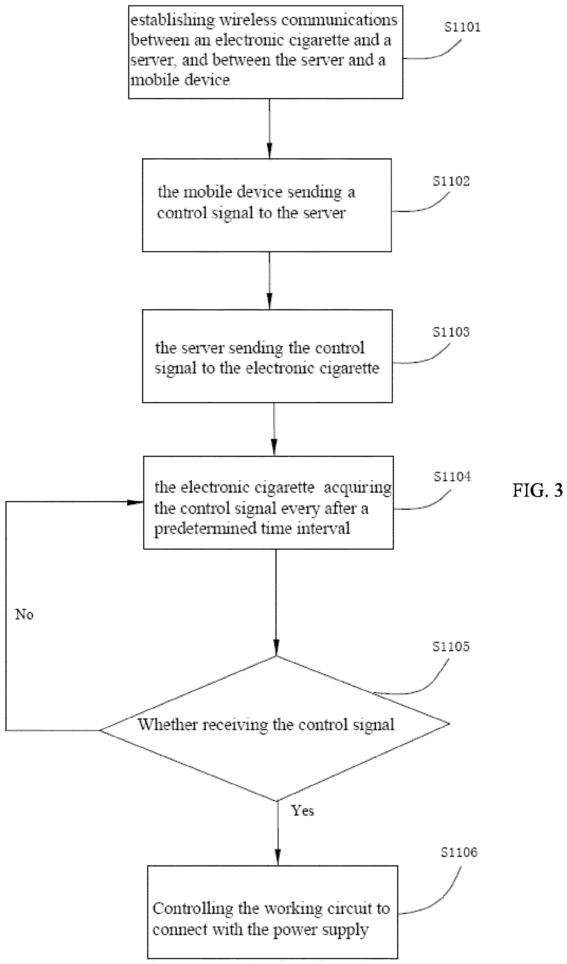

The present disclosure relates to a control method of an electronic cigarette. Referring to FIGS. 1-3, the control method includes the following steps:

In step S1101, wireless communications are established between an electronic cigarette 10 and a server 30, and the server 30 and a mobile device 20.

In step S1102, the mobile device 20 sends a control signal to the server 30.

In step S1103, the server 30 receives the control signal, and sends the control signal to the electronic cigarette 10.

In step S1104, the electronic cigarette 10 acquires the control signal sent by the server 30, every after a predetermined time interval. In the present embodiment, the predetermined time interval is about 0.2 seconds.

In step S1105, the electronic cigarette 10 judges whether receiving the control signal. If the electronic cigarette 10 receives the control signal, step S1106 is executed; else, returning to the step S1106.

In step S1106, based on the control signal, the electronic cigarette 10 controls a working circuit of an atomizing element 122 to connect with a power supply 112. The atomizing element 122 is configured for atomizing tobacco liquid to form aerosol.

In the present embodiment, when the electronic cigarette 10 receives the control signal, the electronic cigarette 10 controls the working circuit 101 to connect with the power supply 112.

In the present embodiment, the wireless communication connection between the electronic cigarette 10 and the mobile device 20 may be Bluetooth, infrared, Near Field Communication (NFC), or Wi-Fi. The mobile device 20 may be a cell phone, or a laptop computer.

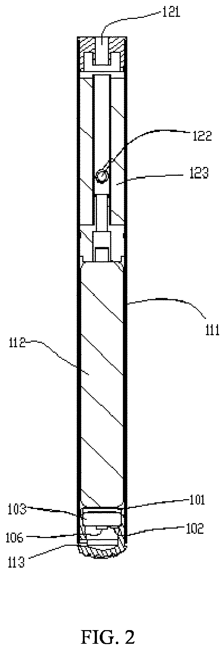

Referring to FIG. 2, in the present embodiment, the working circuit 101 further includes a switch 103, and the switch 103 is an electronic air actuating switch. The switch 103 is configured for enabling or disenabling the atomizing element 122.

In the present embodiment, in case a child gets the electronic cigarette 10, he/she needs to get the mobile device 20 if he/she wants to use the electronic cigarette 10. Furthermore, he/she should use the mobile device 20 to send the control signal for unlocking. Accordingly, it is effective to prevent children from using the electronic cigarette 10.

Referring to FIGS. 1-2, a control system is shown. The control system includes a server 30, a mobile device 20, and an electronic cigarette 10. The electronic cigarette 10 includes a housing 111, a power supply 112, a control unit 106, tobacco liquid 123, an atomizing element 122, a working circuit 101, and a switch 103. The power supply 112, the control unit 106, tobacco liquid 123, the atomizing element 122, the working circuit 101, and the switch 103 are received in the housing 111. The atomizing element 122 is electrically connected to the working circuit 101. The switch 103 is connected to the working circuit 101. The switch 103 is configured for activating the atomizing element 122 when the working circuit 101 is connected to the power supply 112.

Referring to FIG. 2, the housing 111 defines an air inlet 113 and an air outlet 121 at two opposite ends. The control unit 106 is configured for receiving a control signal, and controlling the working circuit 101 to connect with the power supply 112 based on the control signal. The control unit 106 includes a wireless communication element 102. The wireless communication element 102 is in communication with the server 30, and configured for receiving the control signal. The mobile device 20 sends a control signal to the server 30. The server 30 is configured for receiving and sending the control signal to the electronic cigarette 10, so that the control unit 106 controls the working circuit 101 to connect with the power supply 112.

In the present embodiment, the switch 103 is an electronic air actuating switch.

In the present embodiment, the wireless communication connection between the electronic cigarette 10 and the mobile device 20 may be Bluetooth, infrared, Near Field Communication (NFC), or Wi-Fi. Correspondingly, the wireless communication element 102 may be a Bluetooth device, an infrared device, an NFC device, or a Wi-Fi device.

The mobile device 20 may be a cell phone, or a laptop computer. When the wireless communication element 102 receives a control signal, the control unit 106 controls the working circuit 101 to connect with the power supply 112.

It is understood that the above-described embodiments are intended to illustrate rather than limit the disclosure. Variations may be made to the embodiments and methods without departing from the spirit of the disclosure. Accordingly, it is appropriate that the appended claims be construed broadly and in a manner consistent with the scope of the disclosure.

## Claims

1. A control method of an electronic cigarette, the electronic cigarette comprising an atomizing element, a working circuit, and a power supply, the working circuit being electrically connected to the atomizing element, the control method comprising:
(a) a mobile device sending a control signal to a server;
(b) the server receiving and sending the control signal to the electronic cigarette; and
(c) the electronic cigarette receiving the control signal and controlling the working circuit to connect with the power supply based on the control signal.

2. The control method according to claim 1, further comprising following steps between the steps (b) and (c):
(d) the electronic cigarette acquiring the control signal every after a predetermined time interval;
(e) if the electronic cigarette receives the control signal, the electronic cigarette controlling the working circuit to connect with the power supply based on the control signal; if not receiving the control signal, returning to step (d).

3. The control method according to claim 1, wherein the mobile device and the server are in communication via wireless communication.

4. The control method according to claim 3, wherein the wireless communication connection is achieved by Bluetooth, infrared, NFC, or Wi-Fi.

5. The control method according to claim 1, wherein the electronic cigarette and the server are in communication via wireless communication.

6. The control method according to claim 3, wherein the wireless communication connection is achieved by Bluetooth, infrared, NFC, or Wi-Fi.

7. A control system of an electronic cigarette, comprising:
a mobile device;
a server;
an electronic cigarette, the electronic cigarette comprising:
an atomizing element configured for atomizing tobacco liquid;
a power supply;
a working circuit electrically connecting to the atomizing element;
a control unit;
wherein the mobile device is configured for sending a control signal to the server, the server is configured for receiving the control signal and sending the control signal to the electronic cigarette, the control unit is configured for receiving the control signal, and controlling the working circuit to connect to the power supply based on the control signal.

8. The control system according to claim 7, wherein the mobile device is a cell phone or a laptop computer.

9. The control system according to claim 7, wherein the control unit comprises a wireless communication element configured for communicating with the server.

10. The control system according to claim 9, wherein the wireless communication element is a device selecting from a group consisting of a Bluetooth device, an infrared device, an NFC device and a Wi-Fi device.

11. The control system according to claim 7, further comprising an electronic air actuating switch configured for enabling or disenabling the atomizing element.

12. An electronic cigarette, comprising:
an atomizing element configured for atomizing tobacco liquid;
a power supply;
a working circuit electrically connecting to the atomizing element; and
a control unit, the control unit being configured for receiving a control signal, and controlling the working circuit to connect to the power supply based on the control signal.

13. The electronic cigarette according to claim 12, further comprising an electronic air actuating switch configured for enabling or disenabling the atomizing element.

14. The electronic cigarette according to claim 12, wherein the control unit comprises a wireless communication element configured for communicating with the server.

15. The electronic cigarette according to claim 14, wherein the wireless communication element is a device selecting from a group consisting of a Bluetooth device, an infrared device, an NFC device and a Wi-Fi device.
